# EUROPÄISCHE PATENTANMELDUNG

(11) **EP 0 965 362 A1**
(43) Veröffentlichungstag der Anmeldung: **22.12.1999**
(21) Anmeldenummer: 99111816.7
(22) Anmeldetag: 18.06.1999
(51) Int. Cl.: A61N 1/39

(54) **Medizinisches Notfallgerät mit eingebauter Akkumulatoreinrichtung**

(30) Priorität: 19.06.1998 DE 19827478
(71) Anmelder: Marquette Hellige GmbH, 79111 Freiburg i. Br. (DE)
(72) Erfinder: Mikloss, Walther, 79211 Denzlingen (DE); Steffan, Uwe, 79682 Todtmoos (DE)
(74) Vertreter: MÜLLER & HOFFMANN Patentanwälte

(57) **Zusammenfassung**

Die Erfindung betrifft ein medizinisches Notfallgerät mit eingebauter Akkumulatoreinrichtung (1, 2, 3), bei dem die Akkumulatoreinrichtung wenigstens zwei Akkumulatoren (1, 2) umfaßt. Eine Steuereinrichtung entlädt in einem Pflegefunktionsbetrieb die Akkumulatoren (1, 2) nacheinander auf Ladeschlußspannung und lädt diese sodann wieder auf, wobei ein voller Akkumulator immer betriebsbereit gehalten ist.

## Beschreibung

Die vorliegende Erfindung betrifft ein medizinisches Notfallgerät mit eingebauter Akkumulatoreinrichtung.

Medizinische Notfallgeräte mit eingebauter Akkumulatoreinrichtung werden hauptsächlich im Rettungsdienst verwendet. Sie sind dort oft nur kurze Zeit in Betrieb und werden nach dem Einsatz sofort wieder mit dem Netz verbunden, um ihre Akkumulatoreinrichtungen aufzuladen. Mit anderen Worten, medizinische Notfallgeräte mit eingebauter Akkumulatoreinrichtung sind meist nur kurzzeitig im Einsatz, jedoch die überwiegende Zeit am Netz angeschlossen, um auf jeden Fall sicherzustellen, daß die Akkumulatoreinrichtung des Notfallgerätes im Einsatzfall ihre volle Leistung erbringen kann.

Dies ist bei Defibrillatoren von herausragender Bedeutung, da diese im Einsatz voll leistungsfähig sein müssen, um zur Herzschlaganregung ausreichende Spannungs- bzw. Stromstöße liefern zu können.

Nun gibt es aber bei Akkumulatoren und speziell bei Nickel-Cadmium-Akkumulatoren den sogenannten "Memory-Effekt": werden Akkumulatoren wieder aufgeladen, bevor sie vollständig entladen sind, so können sich auf ihrer negativen Elektrode Kristalle bilden. Bei Nickel-Cadmium-Akkumulatoren bilden sich so auf der negativen Elektrode Cadmiumkristalle. Dadurch entsteht in vollkommen unerwünschter Weise eine zweite Entladestufe. Der Akkumulator speichert diese Stufe als Entladestufe für den nächsten Zyklus in seinem "Gedächtnis", obwohl unter dieser Stufe noch Kapazität verfügbar ist. Beim nächsten Entladevorgang erinnert sich der Akkumulator nur noch an diese reduzierte Kapazität. Folgen weitere unvollständige Entladezyklen, so schaukelt sich der Prozeß immer weiter hoch, was bedeutet, daß die Speicherfähigkeit des Akkumulators weiter abnimmt.

Man weiß daher, daß Akkumulatoren hin und wieder vollständig entladen werden sollten, um auf diese Weise den "Memory-Effekt" zu vermeiden und die Lebensdauer des Akkumulators zu verlängern.

Dieser Memory-Effekt wirkt sich bei medizinischen Notfallgeräten in nachteilhafter Weise aus, so daß deren Einsatzfähigkeit nur dadurch gewährleistet werden kann, daß in bestimmten Abständen die Akkumulatoren ausgetauscht werden.

Es ist daher Aufgabe der vorliegenden Erfindung, ein medizinisches Notfallgerät zu schaffen, das trotz des zwangsläufig bei Akkumulatoren vorhandenen Memory-Effektes zuverlässig ständig einsatzbereit ist.

Diese Aufgabe wird bei einem medizinischen Notfallgerät der eingangs genannten Art erfindungsgemäß dadurch gelöst, daß die Akkumulatoreinrichtung wenigstens zwei Akkumulatoren umfaßt und eine Steuereinrichtung vorgesehen ist, die in einem Pflegefunktionsbetrieb die Akkumulatoren nacheinander auf Ladeschlußspannung entlädt und sodann wieder auflädt, wobei mindestens ein voller Akkumulator immer betriebsbereit gehalten ist.

Die Steuereinrichtung beendet in vorteilhafter Weise nach aufeinanderfolgendem Entladen und Laden der Akkumulatoren den Pflegefunktionsbetrieb.

In einer Weiterbildung der Erfindung ist vorgesehen, daß bei einer aus zwei Akkumulatoren bestehenden Akkumulatoreinrichtung die Steuereinrichtung im Pflegefunktionsbetrieb die beiden Akkumulatoren voll auflädt, danach die elektrische Verbindung zum Versorgungsnetz trennt, anschließend durch Gerätebetrieb den ersten Akkumulator bis zur Ladeschlußspannung entlädt, die elektrische Verbindung zum Versorgungsnetz wieder herstellt, den ersten Akkumulator wieder voll auflädt, die elektrische Verbindung zum Versorgungsnetz trennt, den zweiten Akkumulator durch Gerätebetrieb bis zur Ladeschlußspannung entlädt, die elektrische Verbindung zum Versorgungsnetz wieder herstellt, den zweiten Akkumulator wieder voll auflädt und anschließend den Pflegefunktionsbetrieb automatisch beendet.

Die vorliegende Erfindung ist in besonders vorteilhafter Weise bei einem Defibrillator einsetzbar, da bei diesem die Funktionsfähigkeit mit voller Ladespannung von großer Bedeutung ist.

Bei dem erfindungsgemäßen Notfallgerät wird also eine Tiefentladung und Ladung der Akkumulatoren der Akkumulatoreinrichtung programmgesteuert vorgenommen, wobei automatisch dafür gesorgt wird, daß das Notfallgerät ständig einsatzbereit ist. Durch den automatischen Programmablauf wird gewährleistet, daß immer ein Akkumulator voll aufgeladen ist, so daß die volle Verfügbarkeit des Gerätes sichergestellt werden kann. Das Laden und Entladen der jeweiligen Akkumulatoren erfolgt in dem medizinischen Notfallgerät selbst, wobei - bei zwei Akkumulatoren - ein Akkumulator betriebsbereit vorgehalten wird, während der andere Akkumulator "gepflegt" wird.

Der Pflegefunktionsbetrieb kann zu jeder Zeit gestartet werden. Nach Abschluß des Pflegebetriebes hat der Anwender des medizinischen Notfallgerätes wieder wenigstens zwei gewartete, voll geladene Akkumulatoren zur Verfügung. Sollte während des Pflegefunktionsbetriebes selbst das medizinische Notfallgerät benötigt werden, so steht dieses zu jeder Zeit mit wenigstens einem voll geladenen Akkumulator zu Verfügung. Das heißt, das erfindungsgemäße medizinische Notfallgerät ist jedenfalls ständig einsatzbereit.

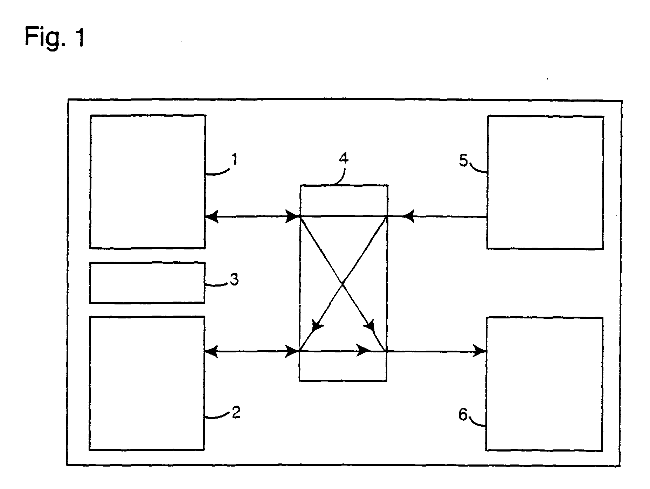

Nachfolgend wird die Erfindung anhand der Zeichnungen näher erläutert, wobei speziell auf den Ablauf des Pflegefunktionsbetriebes eingegangen wird. In der einzigen Figur der Zeichnung ist ein schematisches Blockschaltbild für den Pflegefunktionsbetrieb dargestellt.

Das erfindungsgemäße medizinische Notfallgerät verfügt über wenigstens zwei Akkumulatoren 1, 2, wobei gegebenenfalls noch weitere Akkumulatoren 3 vorgesehen sein können. Außerdem ist eine Steuereinheit 4 vorgesehen, mit der in einem Pflegefunktionsbetrieb ein Laden (vgl. Bezugszeichen 5) und ein Entladen (vgl. Bezugszeichen 6) des Akkumulators 1 bzw. des Akkumulators 2 (oder der weiteren Akkumulatoren 3) vorgenommen werden kann.

Der Ablauf eines Pflegefunktionsbetriebes soll nun im folgenden erläutert werden.

Zunächst wird das medizinische Notfallgerät am Netz angeschlossen und eingeschaltet. Sodann erfolgt die Anwahl des Pflegefunktionsbetriebes, was automatisch (in vorbestimmten Zeitabständen) oder manuell geschehen kann. Ist der Pflegefunktionsbetrieb ausgewählt, was durch Eingabe eines entsprechenden Ansteuersignales an die Steuereinrichtung 4 erfolgt, werden beide Akkumulatoren 1 und 2 aufgeladen, so daß das medizinische Notfallgerät, das mit diesen Akkumulatoren ausgestattet ist, voll verfügbar ist. Sodann wird die Verbindung zum Netz unterbrochen. Der Akkumulator 1 (oder der Akkumulator 2) wird sodann durch Gerätebetrieb bis zur Ladeschlußspannung entladen. Anschließend wird die Verbindung zum Netz wieder hergestellt. Nach Herstellung der Netzverbindung wird der Akkumulator 1 (oder der Akkumulator 2) wieder voll aufgeladen. Es erfolgt sodann eine Umschaltung des Gerätebetriebes auf den anderen Akkumulator, im vorliegenden Beispiel also auf den Akkumulator 2. Die Verbindung zum Netz wird anschließend getrennt. Sodann wird der Akkumulator 2 durch Gerätebetrieb bis zur Ladeschlußspannung entladen. Nach Entladung des Akkumulators 2 wird die Verbindung zum Netz wieder hergestellt. Sodann wird der Akkumulator 2 wieder voll aufgeladen. Damit sind beide Akkumulatoren 1, 2 nach diesem "Pflegefunktionsbetrieb" wieder voll betriebsfähig.

Die Erfindung stellt so ein medizinisches Notfallgerät zur Verfügung, bei dem mit Hilfe des Pflegefunktionsbetriebes gewährleistet ist, daß die Akkumulatoren immer ihre volle Leistung zu erbringen vermögen. Nachteile, die bei üblichen Notfallgeräten durch den "Memory-Effekt" bedingt sind, werden bei dem erfindungsgemäßen medizinischen Notfallgerät zuverlässig vermieden.

## Patentansprüche

1. Medizinisches Notfallgerät mit eingebauter Akkumulatoreinrichtung (1, 2, 3),
dadurch gekennzeichnet, daß
die Akkumulatoreinrichtung (1, 2, 3) wenigstens zwei Akkumulatoren (1, 2) umfaßt, und
eine Steuereinrichtung (4) vorgesehen ist, die in einem Pflegefunktionsbetrieb die Akkumulatoren (1, 2) nacheinander auf Ladeschlußspannung entlädt und sodann wieder auflädt, wobei mindestens ein voller Akkumulator (1 bzw. 2) immer betriebsbereit gehalten ist.

2. Medizinisches Notfallgerät nach Anspruch 1, dadurch gekennzeichnet, daß die Steuereinrichtung (4) nach aufeinanderfolgendem Entladen und Laden der Akkumulatoren (1, 2) den Pflegefunktionsbetrieb beendet.

3. Medizinisches Notfallgerät nach Anspruch 1, dadurch gekennzeichnet, daß bei einer aus zwei Akkumulatoren (1, 2) bestehende Akkumulatoreinrichtung (1, 2, 3) die Steuereinrichtung (4) im Pflegefunktionsbetrieb die beiden Akkumulatoren (1, 2) voll auflädt, danach die elektrische Verbindung zum Versorgungsnetz trennt, anschließend durch Gerätebetrieb den ersten Akkumulator (1) bis zur Ladeschlußspannung entlädt, die elektrische Verbindung zum Versorgungsnetz wieder herstellt, den ersten Akkumulator (1) wieder voll auflädt, die elektrische Verbindung zum Versorgungsnetz trennt, den zweiten Akkumulator (2) durch Gerätebetrieb bis zur Ladeschlußspannung entlädt, die elektrische Verbindung zum Versorgungsnetz wieder herstellt, den zweiten Akkumulator (2) wieder voll auflädt und anschließend den Pflegefunktionsbetrieb automatisch beendet.

4. Medizinisches Notfallgerät nach einem der Ansprüche 1 bis 3, dadurch gekennzeichnet, daß es ein Defibrillator ist.

5. Defibrillator mit eingebauter Akkumulatoreinrichtung (1, 2, 3) aus zwei Akkumulatoren (1, 2) und einer Steuereinrichtung (4), die in einem Pflegefunktionsbetrieb die beiden Akkumulatoren (1, 2) voll auflädt, danach die elektrische Verbindung zu einem Versorgungsnetz trennt, anschließend durch Gerätebetrieb den ersten Akkumulator (1) bis zur Ladeschlussspannung entlädt, die elektrische Verbindung zum Versorgungsnetz wieder herstellt, den ersten Akkumulator (1) wieder voll auflädt, die elektrische Verbindung zum Versorgungsnetz trennt, den zweiten Akkumulator (2) durch Gerätebetrieb bis zur Ladeschlussspannung entlädt, die elektrische Verbindung zum Versorgungsnetz wieder herstellt, den zweiten Akkumulator (2) wieder voll auflädt und anschließend den Pflegefunktionsbetrieb automatisch beendet.
